# EUROPEAN PATENT SPECIFICATION

(11) **EP 1 100 870 B1**
(45) Date of publication and mention of the grant of the patent: **02.01.2008**
(21) Application number: 99941984.9
(22) Date of filing: 29.07.1999
(51) Int. Cl.: C12N 5/02, C12N 5/08, A61K 45/00, A61P 9/00, A61P 9/04

(54) **IMPROVEMENT OF CARDIAC FUNCTION BY MESENCHYMAL STEM CELL TRANSPLANTATION**
VERBESSERUNG DER HERZFUNKTION DURCH TRANSPLANTATION MESENCHYMALER STAMMZELLEN
AMELIORATION DU FONCTIONNEMENT CARDIAQUE PAR TRANSPLANTATION DE CELLULES SOUCHES MESENCHYMATEUSES

(30) Priority: 31.07.1998 US 94794 P; 14.04.1999 US 129152 P
(43) Date of publication of application: 23.05.2001
(60) Divisional application: 07119782.6
(73) Proprietor: GENZYME CORPORATION, Cambridge, MA 02138 (US)
(72) Inventor: MICKLE, Donald, A., G., Grand Valley, Ontario, LON 1GO (CA); LE, Ren-Ke, Scarborough, Ontario M1V 1B4 (CA); WEISEL, Richard, D., Toronto, Ontario M4X 1S8 (CA)
(74) Representative: Adams, Harvey Vaughan John
(86) International application number: PCT/US1999/017326
(87) International publication number: WO 2000/006701

(56) References cited:
- WO-A-99/03973
- WO-A-99/49015
- US-A- 5 197 985
- US-A- 5 486 359
- TOMITA S ET AL: "AUTOTRANSPLANTED MESENCHYMAL STEM CELLS IMPROVE FUNCTION AFTER A MYOCARDIAL INFARCTION" CIRCULATION, AMERICAN HEART ASSOCIATION, DALLAS, TX, US, vol. 98, no. 17, SUPPL, 27 October 1998 (1998-10-27), pages I-200, XP009032355 ISSN: 0009-7322
- MAKINO S ET AL: "CARDIOMYOCYTES CAN BE GENERATED FROM MARROW STROMAL CELL IN VITRO" JOURNAL OF CLINICAL INVESTIGATION, NEW YORK, NY, US, vol. 103, no. 5, March 1999 (1999-03), pages 697-705, XP000990317 ISSN: 0021-9738
- WAKITANI S ET AL: "MYOGENIC CELLS DERIVED FROM RAT BONE MARROW MESENCHYMAL STEM CELLS EXPOSED TO 5-AZACYTIDINE" MUSCLE & NERVE, NEW YORK, NY, US, vol. 18, no. 12, December 1995 (1995-12), pages 1417-1426, XP009006910
- FERRARI G ET AL: "Muscle regeneration by bone marrow -derived myogenic progenitors [see comments] [published erratum appears in Science 1998 Aug 14;281(5379):923]" SCIENCE, AMERICAN ASSOCIATION FOR THE ADVANCEMENT OF SCIENCE,, US, vol. 279, 6 March 1998 (1998-03-06), pages 1528-1530, XP002098163 ISSN: 0036-8075

## Description

### Field of the Invention

This invention relates to methods of mesenchymal stern cell preparation and transplantation of the cell preparation into diseased or scarred myocardium to improve cardiac function.

### Background of the Invention

During the late stages of heart disease, cardiomyocytes die in response to physiological stress. Because lost cardiomyocytes are not replaced in adult myocardial tissue, the remaining cardiomyocytes are placed under yet further stress, thereby inducing the death of yet more cardiomyocytes and further weakening of the myocardium. Congestive heart failure results when the progressively weakening heart can no longer keep up with the physical demands placed upon it.

To prevent or lessen heart failure in a patient with heart disease, it is necessary to inhibit further loss of cardiac function, and ideally, to improve cardiac function. Because the heart does not replace its cardiomyocytes *in vivo*, one potential approach for improving cardiac function is by transplantation of cells that improve the function of diseased or scarred myocardium: preferably, cells that are elastic, and optimally, contractile.

Allogeneic fetal cardiomyocytes transplanted into normal myocardium or scar tissue have been shown to improve cardiac function. However, a transplantation study in rats with scarred myocardium has demonstrated that allogeneic cells are immunologically rejected and necrosed by 24 weeks after transplantation, despite immunosuppressive therapy (Li et al., Circulation 96(Suppl. II):179-187, 1997). Immunorejection would be avoided by transplantation of autologous cells.

Bone marrow contains multipotential mesenchymal stern cells that are known to differentiate into skeletal myocytes, osteocytes, chondrocytes, and lipocytes *in vitro.* Since mesenchymal stern cells from bone marrow are easily obtained, it would be desirable if these cells could be directed to differentiate into cells that display characteristics of cardiomyocytes and that improve cardiac function after transplantation into diseased or scarred myocardial tissue.

### Summary of the Invention

We have found that mesenchymal stern cells isolated from bone marrow can be used to improve heart function. Mesenchymal stern cells can be induced, by treatment with 5-azacytidine, to differentiate into cardiomyocyte-like cells *in vitro.* Implantation of such 5-azacytidine-treated cells into myocardial scar tissue decreases scar area, increases scar thickness, and improves cardiac function. Moreover, mesenchymal stern cells not pre-treated with 5-azacytidine also differentiate into cardiomyocyte-like cells after transplantation into myocardial tissue, indicating that the *in vivo* cardiac milieu directs cardiogenic differentiation of these cells.

In a first aspect, the invention features a use of a cellular suspension containing mesenchymal stern cells for the preparation of a pharmaceutical composition for treating scarred myocardial tissue, wherein the pharmaceutical composition is to be administered to said scarred myocardial tissue.

In various preferred embodiments of the first aspect of the invention, at least one mesenchymal stern cell has been induced to differentiate into a cardiomyogenic cell; at least one mesenchymal stern cell is to be integrated into a capillary wall in said scarred myocardial tissue; the mesenchymal stern cells have been cultured for at least 7 days; the mesenchymal stern cells have been co-cultured with cardiomyocytes; the mesenchymal stern cells are autologous; or the mesenchymal stern cells are isolated from bone marrow.

In yet other preferred embodiments of the first aspect of the invention, the mesenchymal stern cells have been exposed to 5-azacytidine, or an analog thereof; the 5-azacytidine or analog thereof is present at a concentration of 1, to 100 µM; or the 5-azacytidine or analog thereof is present at a concentration of 10 µM.

In still another preferred embodiment of the first aspect of the invention, the pharmaceutical composition is to be administered by injecting.

In further embodiments of the first aspect of the invention, the treatment improves cardiac function, and the myocardial tissue is within a human.

In a second aspect, the invention features a method of obtaining a population of cells containing cardiomyogenic cells. The method includes: (a) obtaining mesenchymal stern cells; (b) exposing the mesenchymal stern cells to 5-azacytidine or an analog thereof, wherein the exposing is sufficient to obtain at least one cardiomyogenic cell; and (c) placing said cells from step (b) into a suitable medium for injecting the cells into scarred myocardium.

In various preferred embodiments of the second aspect of the invention, at least one mesenchymal stern cell has been induced to differentiate into a cardiomyogenic cell; at least one mesenchymal stern cell has been induced to differentiate into an endothelial cell; the mesenchymal stern cells have been cultured for at least 7 days; the mesenchymal stern cells have been co-cultured with cardiomyocytes; the mesenchymal stern cells are isolated from bone marrow. In other embodiments of the second aspect of the invention, the 5-azacytidine or analog thereof is present at a concentration of 1 to 100 *µ*M; or the 5-azacytidine or analog thereof is present at a concentration of 10*µ*M.

In yet another embodiment of the second aspect of the invention, the medium is not Iscove's modified Dulbecco's medium (IMDM).

In all embodiments of the first two aspects of the invention wherein at least one mesenchymal stern cell has been induced to differentiate into a cardiomyogenic cell or an endothelial cell, or wherein at least one mesenchymal stern cell integrates into a capillary wall, it is preferable that at least 0.5% to 5%; more preferably, at least 5% to 10%; still more preferably, at least 10% to 25%; even more preferably, at least 25% to 50%; yet more preferably, at least 50% to 75%; and most preferably, at least 75% to 90% of the mesenchymal stern cells differentiate into cardiomyogenic or endothelial cells, or integrate into a capillary wall.

Also disclosed herein is a therapeutic composition containing mesenchymal stern cells and a pharmaceutically acceptable carrier appropriate for injection of the cells into damaged or scarred myocardium. Preferably the mesenchymal stern cells have been exposed to 5-azacytidine or an analog thereof. Preferably the pharmaceutically acceptable carrier is not Iscove's modified Dulbecco's medium (IMDM).

In a further embodiment of the aspects of the invention, the mesenchymal stern cells are not passaged, and the mesenchymal stern cells are human mesenchymal stern cells.

5-azacytidine and its analogs are used at a concentration of 1 to 100 µM, for example, 2 to 5 µM, 5 to 10 µM, 10 to 25 µM, 25 to 50 µM, or 50 to 100 µM. Preferably, the 5-azacytidine or 5-azacytidine analog is used at a final concentration of 5 to 25 µM, and, most preferably, at a final concentration of 10 µM.

By "cardiomyogenic cell" is meant a cell that expresses cardiac-specific troponin I and myosin heavy chain encoded by endogenous cellular genes.

By "analog of 5-azacytidine" or "5-azacytidine analog" is meant a compound that, when administered in a sufficient amount, is capable of inducing a mesenchymal stern cell to differentiate into a cardiomyogenic cell. One example of a 5-azacytidine analog is 5-aza-2'-deoxycytidine.

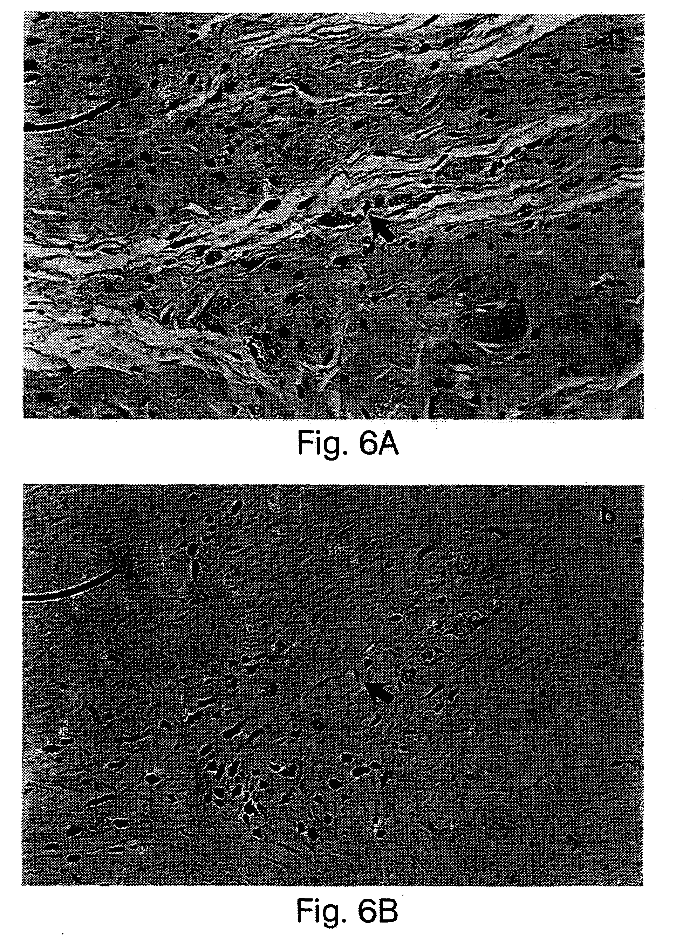

By "integrated into a capillary wall" is meant that a mesenchymal stern cell transplanted in damaged or scarred myocardial tissue is later found incorporated into a capillary in the myocardial tissue, as described in Example II below and shown in Fig. 6B.

By "suitable medium for injecting cells into damaged or scarred myocardium" or "pharmaceutically acceptable carrier appropriate for injection of the cells into damaged or scarred myocardium" is meant a solution that allows optimal survival of mesenchymal stern cells before and after injection of the cells into myocardial or scar tissue, and further, does not have a significantly adverse effect on the injected myocardial tissue, scar tissue, or injected host.

By "passaging" is meant subculturing the cells, for example, detaching (e.g., by trypsin digestion) cultured mesenchymal stern cells that are attached to a cell culture vessel (e.g., a flask or dish), transferring the cells to a second cell culture vessel containing culture medium (e.g., Iscove's modified Dulbecco's medium), and allowing the cells to become attached to the second vessel such that the cells cannot be collected from the second vessel unless trypsin digestion or equivalent means for detachment (i.e., cell scraping) are used.

By "improves cardiac function" is meant that administration of mesenchymal stern cells to damaged or scarred myocardial tissue results in at least one of the following by five weeks after transplantation (preferably by four weeks after transplantation): an increase in capillary density within the region of the transplant of at least 1.5-fold, preferably by at least 2-fold; a decrease in the transmural scar area of at least 5%, more preferably by at least 10%, 15%, 20%, or 25%; an increase in the transmural scar thickness of at least 5%, preferably by at least 10%, 15%, 20%, or 25%; a decrease in the left ventricular chamber volume/body weight ratio of at least 5%, more preferably by at least 10%, 15%, 20%, 25%, or 30%; an increase in systolic pressure or developed pressure of at least 5%, more preferably by at least 10% or 15%; or an increase in ejection fraction by at least 5%, preferably by at least 10%, 15%, 20%, or 25%.

### Brief Description of the Drawings

Fig. 1 is a representation of a photomicrograph showing cultured mesenchymal stern cells (MSCs) 7 days after isolation from bone marrow.
Fig. 2A is a representation of a photomicrograph showing cultured MSCs 10 days after isolation from bone marrow, which were treated with 5-azacytidine on day 3 (magnification is 200X).
Fig. 2B is a representation of a photomicrograph showing cultured MSCs 21 days after isolation from bone marrow (cells were treated with 5-azacytidine on day 3 and immunofluorescently stained on day 21; magnification is 400X).
Fig. 3 is a representation of a photomicrograph showing transplanted BrdU-labeled MSCs in myocardial scar tissue (magnification is 400X).
Figs. 4A-4B are representations of photomicrographs showing transplanted BrdU-labeled MSCs in myocardial scar tissue stained with hematoxylin and eosin (Fig. 4A) or an antibody against cardiac-specific troponin I (Fig. 4B); (magnification is 200X).
Fig. 5 is a graph showing the capillary densities in myocardial scar tissue transplanted with fresh MSCs, cultured MSCs, 5-azacytidine-treated cultured MSCs, or mock-trasplanted (negative control).
Figs. 6A-6B are representations of photomicrographs showing that transplanted bone-marrow-derived endothelial cells are integrated into capillary walls in myocardial scar tissue stained with hematoxylin and eosin (Fig. 6A) or an antibody against BrdU (Fig. 6B).
Fig. 7 is a graph showing the relative areas of myocardial scars transplanted with fresh MSCs, cultured MSCs, 5-azacytidine-treated cultured MSCs, or mock-transplanted (negative control).
Fig. 8 is a graph showing the relative thicknesses of myocardial scars transplanted with fresh MSCs, cultured MSCs, 5-azacytidine-treated cultured MSCs, or mock-transplanted.
Fig. 9 is a graph showing the ratio of left ventricular chamber size/body weight in rats having myocardial scars and transplanted with fresh MSCs, cultured MSCs, 5-azacytidine-treated cultured MSCs, or mock-transplanted.
Fig. 10A-10C are graphs showing, respectively, systolic, diastolic, and developed pressures in rats having myocardial scars and transplanted with fresh MSCs, cultured MSCs, or 5-azacytidine-treated cultured MSCs, or mock-trasplanted.
Fig. 11 is a diagram showing MIBI scans of myocardial function of a control swine heart at 4 weeks (i.e., pre-mock-transplantation) and 8 weeks (i.e., post-mock-transplantion) after induction of infarction.
Figs. 12A-12B are diagrams showing MIBI scans of myocardial function of an experimental swine heart at 4 weeks (i.e., pre-transplantation) and 8 weeks (i.e., post-transplantion) after induction of infarction.

### Detailed Description of the Invention

In an effort to identify a plentiful source of autologous cells that could improve cardiac function after implantation into a diseased heart, we evaluated cardiogenic differentiation of mesenchymal stern cells (MSCs) isolated from bone marrow, their survival after implantation into myocardial scars, and the effect of the implanted cells on heart function.

As described in further detail below, *in vitro* studies showed that 5-azacytidine (5-aza) induced MSCs to form muscle-like cells that stained positively for cardiomyocyte-specific troponin I. To assess the cardiogenic differentiation of MSCs *in vivo,* three weeks after cryoinjury of the myocardial free wall in rats, freshly isolated MSCs, cultured MSCs, 5-aza-induced MSCs, or culture medium alone (control) were autologously transplanted into the myocardial scar tissue. Cardiac-like muscle cells were observed in all MSC-transplanted scar tissue. The 5-aza-treated MSC transplants improved cardiac function, as indicated by improved developed and systolic pressures of the 5-aza-treated MSC-transplanted group (p<0.05), compared to the other groups. In addition, transplantation of MSCs induced angiogenesis within the transplant area. Improvement of cardiac function by autologous MSC transplantation was also observed in a swine model of myocardial infarction.

We found that the cardiac milieu effect played a significant role in directing cardiomyogenic differentiation of MSCs *in vivo.* MSCs cultured for 7 days in the absence of 5-aza do not differentiate into myogenic cells *in vitro.* In contrast, freshly isolated MSCs, MSCs cultured for 7 days in the absence of 5-aza, or MSCs cultured with 5-aza all differentiated into myogenic-like cells within the transplant. Although 5-aza induces MSCs to differentiate into adipocytes (and such cells were often present in MSC cultures treated with 5-aza), fat tissue was not found in the transplanted area. These observations indicate that, in addition to 5-aza, *in vivo* cardiomyogenic factors play an important role in inducing the differentiation of immature MSCs to cardiomyocyte-like cells.

The following examples will assist those in skilled in the pertinent arts to better understand the invention and its principles and advantages. The following Examples are intended to illustrate the invention, not to limit the scope thereof.

### Example I: Cardiomyogenic Differentiation of Rat Mesenchymal Stern Cells In Vitro

### Methods

### Preparation of Mesenchymal Stern Cells

An incision was made in the hindlimb of each rat while the rat was under general anesthesia, An 18-gauge needle was punctured to the bone and used to aspirate bone marrow (1 ml) from the tibia. The marrow aspirate was transferred to sterile tubes, which contained 10 ml Iscove's modified Dulbecco medium (IMDM; Gibco Laboratories, Gaithersberg, MD), containing antibiotics (penicillin G, 100 U/ml; streptomycin, 100 µg/ml) and 1000 IU heparin. The marrow cell suspension was dispersed into single cells using a 23-gauge needle. The tube was spun at 2000 rpm for 5 min to pellet the cells. After removing the supernatant and fat layer, the cell pellet was re-suspended with IMDM. To separate bone marrow cells and red blood cells, the density centrifugation method described by Yablonka-Reuveni and Nameroff (Histochemistry, 87:27-38, 1987) was used. First, the cell suspension was loaded onto 20% and 60% Percoll (Sigma Chemical Co., St. Louis, MO) layers, which were piled up in a tube, and the tube was centrifuged at 14000 rpm for 10 minutes. The top two-thirds of the total volume were transferred into a tube (a preliminary study showed that these layers contained most of the MSCs). The cells were centrifuged at 2,000 rpm for 10 minutes and then washed with phosphate-buffered saline (PBS) to remove the Percoll. This was repeated and then the cell pellet was resuspended in culture medium and used for *in vitro* and *in vivo* studies.

To identify the optimal conditions for inducing mesenchymal stern cell (MSC) differentiation by 5-azacytidine, the cells were cultured in IMDM containing 10% fetal bovine serum and antibiotics, with 5-azacytidine (5-aza : 0.1, 1, 5, 10, 20, and 100 µM; n=6 for each group), with insulin (1 nM; n=6), or with TGFβ1 (10ng/ml; n=6). The cells were incubated with these agents for 24 hours and then washed with PBS. The medium was changed twice a week for 21 days. Almost all the hematopoietic stern cells were washed away with the medium changes.

To test the effect of cell passaging on cardiomyogenic differentiation of MSCs, samples of MSCs, prepared as described above, were passaged once prior to preparing the cell cultures described below. In this case, the term passaging refers to the distribution of cultured cells growing in a culture dish into new dishes after the cells reach confluence (i.e. when the cells contact each other and cover the surface of the dish).

### Preparation of MSC Cultures for Detection of Cardiac-Specific Contractile Proteins

One group of the MSCs, prepared as above, was, in turn, divided into two groups: A (control) and B, each group having 6 samples. Cells of the control group, A, were cultured for 7 days in IMDM containing 10% fetal bovine serum and antibiotics. The MSCs of group B were initially cultured for 2 days in the above medium after which 5-aza (10 µM) was added to the culture medium. After a 24 hour exposure to 5-aza, the compound was washed out of the culture and the cells were culturing in the medium described above for 4 days more.

### Detection of Cardiac Contractile Proteins in Cultured MSCs

Following the above culturing period, the cells from both groups were stained by immunocytofluorescence to detect the presence of cardiac-specific troponin I (a contractile apparatus-specific protein found only in cardiomyocytes) or muscle-specific myosin heavy chain. Briefly, the staining method entailed washing the cells with phosphate-buffer saline (PBS), followed by incubation in methanol at -20°C for 20 minutes. The dishes were then washed with PBS three times. Monoclonal antibodies specific against cardiac troponin I (Spectral Diagnostic, Toronto) or against myosin heavy chain (Biogenesis, USA), were added to the cells and the dishes were incubated at 37°C for 1 hour. To remove unbound antibody, the dishes were gently shaken at room temperature and washed three times with PBS. A rabbit antibody conjugated with FITC (fluorescein isothiocyanate) against mouse IgG was added to the dishes, after which incubation and rinsing with PBS were performed as described above for the first antibody. Observations were made and photos were taken.

### Detection of Cardiac Contractile Proteins in Passaged Cells

Staining for cardiac-specific troponin I and myosin heavy chain, as described above, was also carried out on MSCs that were first cultured to confluency without 5-aza and then sub-cultured for 24 hours (i.e., passaged one time). Following this, 5-aza (10 µM) was added and the cells were cultured for a further 24 hours. The 5-aza was then washed out and the cells were cultured for 4 days, after which the cells were stained for cardiac-specific troponin I and myosin heavy chain.

### MSC/Cardiomyocyte Co-Culture

Freshly isolated MSCs from adult rats were mixed and co-cultured with adult rat ventricular cardiomyocytes in IMDM containing 10% fetal bovine serum and antibiotics and without 5-aza. The cardiomyocytes were isolated as described below. As controls, cultures of cardiomyocytes alone and MSCs alone were prepared under the same conditions. Following co-culturing for 10 days, the MSCs were stained for cardiac-specific troponin I and myosin heavy chain as described above.

In order to obtain the cardiomyocytes for the co-culturing experiments, adult rats (Charles River Canada Inc. Quebec, Canada) were anesthetized via an intramuscular injection of ketamine hydrochloride (22 mg/kg body weight) followed by an intraperitoneal injection of sodium pentobarbital (30 mg/kg). The hearts were excised and the myocardial tissue was washed with phosphate-buffered saline (NaCl 136.9 mM, KCl 2.7 mM, Na₂HPO₄ 8.1 mM KH₂PO₄ 1.5 mM, pH 7.3). The tissue was minced and incubated in 10 ml PBS containing 0.2% trypsin, 0.1 % collagenase, and 0.02% glucose for 30 minutes at 37°C. The cardiomyocytes were then isolated by repetitive pipetting of the digested myocardial tissue. The cells in the supernatant were transferred into a tube containing 20 ml of cell culture medium (Iscove's modified Dulbecco's medium containing 10% fetal bovine serum, 0.1 mmole/L β-mercaptoethanol, 100 units/ml penicillin and 100 µg/ml streptomycin). The tube was centrifuged at 600 x g for 5 minutes at room temperature and the cell pellet was re-suspended in the cell culture medium for purification.

### Results

### Optimization of MSC Cardiogenesis Induced by 5-Azacytidine

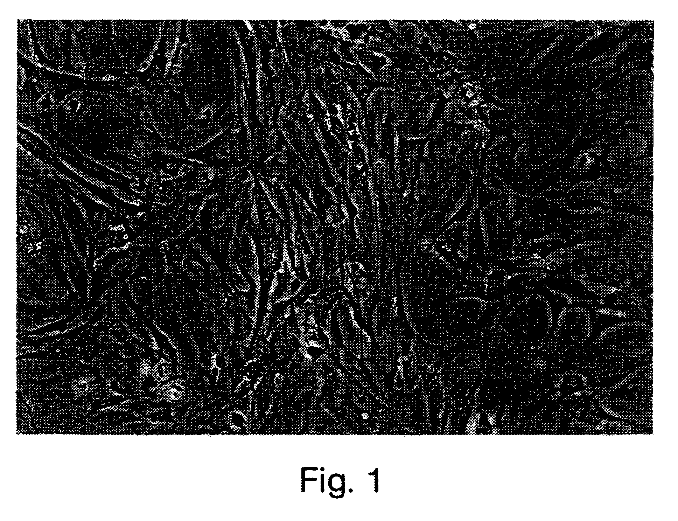

Two major types of cells, mesenchymal stern cells (MSCs) and hematopoietic stern cells, were isolated from Percoll gradients of bone marrow cells. The MSCs were spindle-shaped, attached to the culture dish tightly and proliferated in the culture medium. The hematopoietic stern cells were round-shaped, did not attach to culture dish, and were washed away with the culture medium changes. Fig. 1 shows a photomicrograph of cultured bone marrow cells from Percoll gradients on day 7; almost all cells in the culture dish were spindle-shaped MSCs.

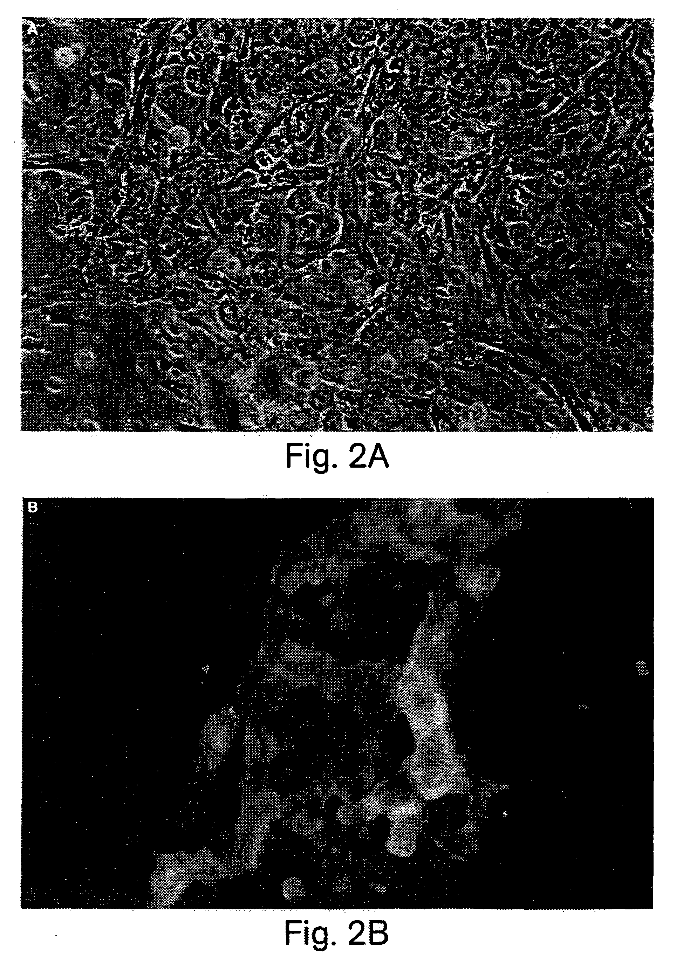

When 5-aza was added to bone marrow cells on the third day of culture, the cells formed multinucleated myotubes on the 10th day of culture (Fig. 2A). 5-aza-treated MSCs stained positively for cardiac specific troponin I (Fig. 2B) and myosin heavy chain on the 21st day. Purified MSCs, cultured with TGFβ and insulin or the medium did not form myotubes and were negative for troponin I and myosin heavy chain.

The optimal conditions for differentiation of MSCs into muscle cells were evaluated by incubating MSCs with 0.1, 1, 5, 10, 20, and 100 µM 5-aza. MSCs incubated with 5-aza at 20 µM and 100 µM appeared abnormal; more than 50% of the cells were necrotic. Although damaged MSCs were also observed in cultures incubated with either 5 µM or 10 µM 5-aza for 24 hours, myotube formation was present. The number of myotubes resulting from incubation with 10 µM 5-aza was visually determined to be greater than the number resulting from incubation with 5 µM 5-aza. Cells cultured with concentrations of 5-aza that were lower than 5 µM 5-aza were morphologically similar to the control cells.

To determine the optimal 5-aza addition time, we compared cultures of MSCs that had been incubated with 10 µM 5-aza on the first, second, or third day after isolation. The number of MSCs in cultures in which 5-aza was added on the first or second day was much smaller than that of cultures in which 5-aza was added on the third day.

As described above, induction of cardiomyogenesis by 5-azacytidine was also attempted using MSCs that were passaged once. It was found that, unlike non-passaged cells, cells that were passaged once did not differentiate into cardiomyocyte-like cells, as indicated by the lack of myotube formation (determined by immunofluorescent staining as described above).

### MSC Cardiomyogenesis After Co-Culture

Qualitative analysis of the cell samples from the co-culturing experiments indicated that MSCs co-cultured with the ventricular cardiomyocytes formed many more myotubes than those cultured with 5-azacytidine. MSCs or cardiomyocytes did not form any myotubes when cultured alone.

### Conclusions

From the above results, we conclude that MSCs can be directed to differentiate into cardiomyocyte-like cells by culturing the mesenchymal stern cells with 5-azacytidine. From these results, it can reasonably be predicted that differentiation of MSCs into cardiomyogenic cells can also be achieved by culturing the cells as described above with analogs of 5-aza such as 5-aza-2'-deoxycytidine.

In addition, co-culturing MSCs with cardiomyocytes results in the differentiation MCS into cardiomyocyte-like cells, as evidenced by the formation of myotubes. Although the cardiomyocytes used in the present experiments were derived from ventricular tissue, it can reasonably be predicted that atrial cardiomyocytes would also have a similar effect on MSCs.

### Example II: Autologous Transplantion of Bone Marrow Cells Improves Cardiac Function in Rats with Myocardial Scars

### Methods

### Animals

Sprague-Dawley rats (Charles River Canada Inc, Quebec, Canada) were used. Male rats, weighing 400 g to 450 g served as both recipients and donors. The rats were anesthetized with intramuscular administration of ketamine hydrochloride (22 mg/kg) followed by intraperitoneal injection of sodium pentobarbital (30 mg/kg). The anesthetized rats were intubated, and positive pressure ventilation was maintained with room air supplemented with oxygen (2 L/min) using a Harvard ventilator (model 683). The rats were monitored for 4 hours postoperatively. Penlong XL (penicillin G benzathine, 150000 U/ml and penicillin G procaine, 15000 U/ml) was given intramuscularly (0.4 ml per rat).

### Generation of Myocardial Scars

Under general anesthesia, adult rat hearts was exposed through a 2-cm left lateral thoracotomy. Cryoinjury was produced with a metal probe 8- 10 mm in diameter, cooled to -190°C by immersion in liquid nitrogen, and applied to the left ventricle free wall (LVFW) for 15 seconds. The procedure was repeated five times, following which a similarly cooled probe was applied ten times for 1 minute periods. The muscle layer and skin incision were closed with 3-0 silk sutures.

### Preparation of Cells for Transplantation

Three weeks after myocardial damage, the rats were randomly divided into four groups. Group 1 (n=9): MSCs freshly prepared, as described above, were resuspended in IMDM and transplanted by injecting into the center of the scar tissue. Group 2 (n=9): MSCs were cultured for 7 days before transplantation. Group 3 (n=12): MSCs were cultured for a total 7 days. 5-aza (10 µM) was added on the third day and incubated with cells for 24 hours. Group 4 (n=12): IMDM was injected as the control.

The cultured cells were dissociated from the culture dishes with 0.05% trypsin (Gibco BRL, Grand Island, NY), neutralized with culture medium and collected by centrifugation at 2,000 rpm for 5 minutes at room temperature. The cells were suspended in IMDM at concentration of 10⁶ cells in 50 µl for transplantation.

It should be noted that we chose a 7 day culturing period as the optimal time period. However, various other culturing periods will be apparent to those skilled in the art.

### Cell Transplantation

Three weeks after cryoinjury, the rat hearts were exposed through a midline sternotomy under general anesthesia. The mesenchymal stern cell (MSC) suspensions of Groups 1, 2 and 3 were injected without leakage into the centers of the myocardial scar tissue using a tuberculin syringe (10⁶ cells in 50 µl). For the control animals, the culture medium of Group 4 above was transplanted into the scar tissue. The chests were closed with 3-0 silk sutures. Antibiotics and analgesics were given as previously described.

### Heart Function Measurements

Five weeks after transplantation, the animals were anesthetized with ketamine and phentobarbital as previously described. A midline sternotomy was performed, the hearts were removed, and the animals were euthanized by exsanguination. Heart function in the four groups was measured using a Langendorff apparatus, which is commonly known in the art, with filtered Krebs-Henseleit buffer (having the composition shown below) equilibrated with 5% CO, and 95% O₂.

### Krebs-Henseleit buffer (pH 7.4):

| Component | Concentration (mmol/L) |
|---|---|
| NaCl | 118.0 |
| KCl | 4.7 |
| KH₂PO₄ | 1.2 |
| CaCl₂ | 2.5 |
| MgSO₄ | 1.2 |
| NaHCO₃ | 25.0 |
| Glucose | 11.0 |

Latex balloons were passed into the left ventricles through the mitral valves and connected to a pressure transducer (model p10EZ; Viggo-Spectramed, Oxnard, CA) and a transducer amplifier and differentiater amplifier (model 11-G4113-01; Gould Instrument System Inc, Valley View, OH). After 30 minutes of stabilization, the coronary flow of the hearts was measured in triplicate by timed collection in the empty beating state. The balloon sizes were increased in 0.02 ml increments from 0.04 ml until LV (left ventricular) edp (end diastolic pressure) reached 30 mmHg by the addition of saline solution. The systolic and diastolic pressures were recorded at each balloon volume, and the developed pressure was calculated as the difference between the systolic and diastolic pressures. The hearts were weighed and the sizes were measured by water displacement.

### Histology

Tissue samples (0.5 cm³) at the transplantation sites were collected 5 weeks after transplantation and fixed in 5% glacial acetic acid in methanol for histological studies. The samples were embedded and sectioned to yield 10 µM slices, which were stained with hematoxylin and eosin as described by the manufacturer (Sigma Chemical Co., St. Louis, MO) The samples were then stained with an antibody against cardiac beta myosin heavy chain.

### Identification of Transplanted MSCs in Myocardial Scars

Myocardial scars were induced in rats under general anaesthesia. Two weeks later bone marrow was aspirated from the rats. The MSCs were cultured and induced with 5-aza as described above, after which the cells were labeled with bromodeoxyuridine (BrdU; Sigma Chemical Co., St. Louis, MO) to identify the transplanted cells within the scar tissue. Briefly, 10 µl of BrdU solution (BrdU 50 mg, dimethyl sulfoxide 0.8 ml, water 1.2 ml) was added into each culture dish on the sixth day of culture and incubated with the cells for 24 hours. Labeling efficiency was about 75%. The labeled cells were transplanted into the scars at 3 weeks after myocardial injury and samples were collected at 5 weeks after transplantation.

Monoclonal antibodies against BrdU were used to localize the transplanted bone marrow cells (Magaud et al. J. Histochem. Cytochem. 37:1517-1627, 1989). Briefly, samples were serially rehydrated with a series of 100%, 95%, and 70% ethanol after deparaffinization with toluene. Endogenous peroxidase in the sample was blocked using 3% hydrogen peroxide for 10 minutes at room temperature. The samples were treated with pepsin for 5 minutes at 42° C and 2N HCl for 30 minutes at room temperature. After rinsing with PBS three times, the samples were incubated with antibodies against BrdU in a moist chamber for 16 hours at room temperature. Negative control samples were incubated in PBS (without the primary antibodies) under the same conditions. The test and control samples were rinsed with PBS three times (15 minutes each) and then incubated with goat anti-rabbit immunoglobulin G conjugated with peroxidase, at 37° C for 45 minutes. The samples were washed three times (15 minutes each) with PBS and then immersed in diaminobenzidine H₂O₂ (2mg/ml diaminobenzidine, 0.03% H₂O₂ in 0.02ml/L phosphate buffer) solution for 15 minutes. After washing with PBS, the samples were covered with a crystal mount and photographed.

### Measurement of Capillary Density in Myocardial Scars

The number of capillary vessels was counted in the scar tissue of all groups using a light microscope at 400x magnification. Five high-power fields in each scar were randomly selected and the number of capillaries in each was averaged and expressed as the number of capillary vessels/ high-power field (0.2mm²)

### Data Analysis

The data discussed below are expressed as the mean ± the standard error. Statistical Analysis System software was used for all analysis (SAS Institute, Cary, NC). Quantities of myosin heavy chain in two groups were evaluated with a t-test (*p*<0.05). Comparisons of continuous variables between more than two groups were performed by a one-way analysis of variance. If the F ratio was significant from the analysis of variance, a Duncan's multiple-range t-test was employed to specify differences between groups (*p*<0.05).

Functional data were evaluated for the four groups mentioned above by an analysis of covariance using intracavitary balloon volumes as the covariant factors and systolic, diastolic, and developed pressures as the dependent variables. The main effects studied were based on group, volume, and the group x volume interaction. If there was an overall difference in the analysis of covariance, multiple pairwise comparisons were performed to specify which groups were different. Because there were multiple pairwise comparisons, a Bonferroni correction was performed, and the critical α level was set at 0.01 for the analysis of covariance.

### Results

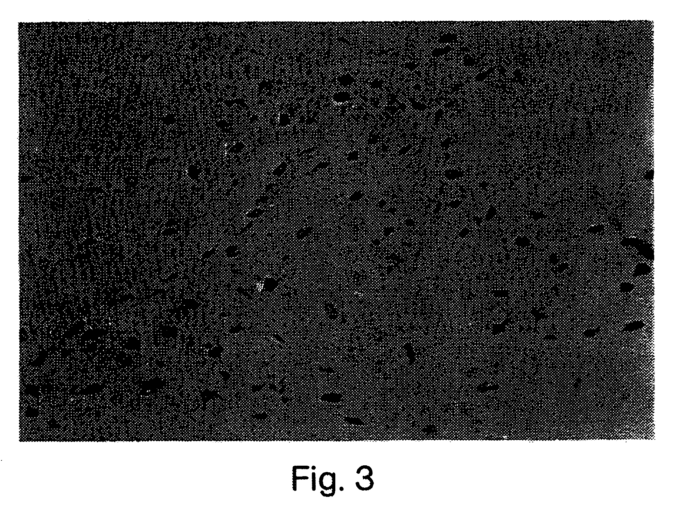

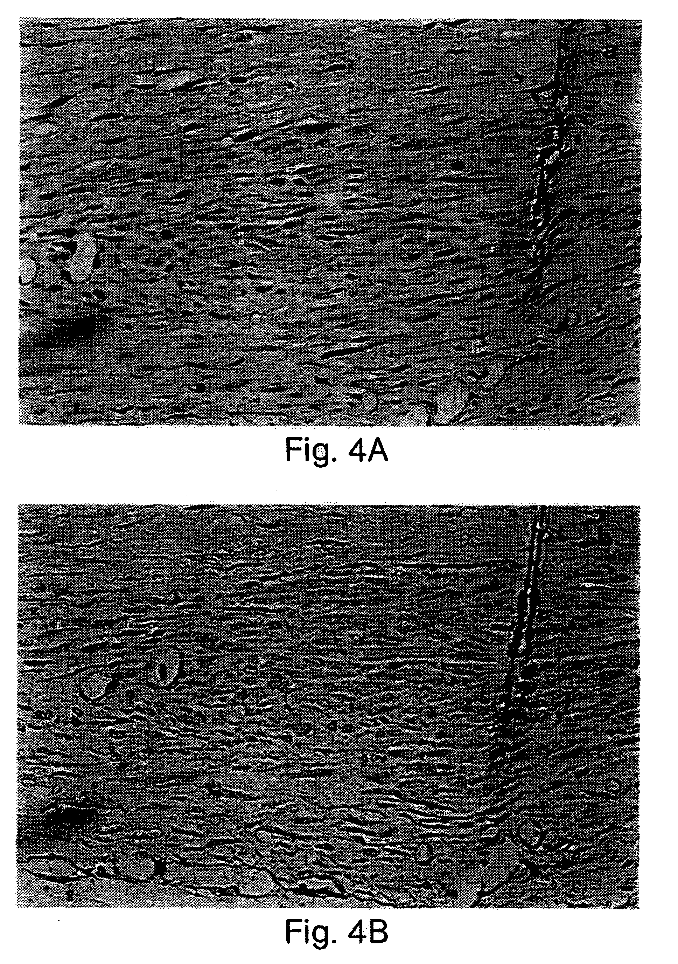

The nuclei of cells that had been treated with 5-aza were labeled with BrdU for 24 hours pre-transplantation. 75.3±4.3% of the cultured cells stained positively. The labeled cells were transplanted into the myocardial scar tissue. At 5 weeks post-transplantation, BrdU-stained cells were observed at the transplanted area (Figs. 3 and 4A). The BrdU-stained cells were muscle-like cells that stained positively for cardiac-specific troponin I (Fig. 4B). Muscle-like cells formed in the scar tissue in the all MSC-transplanted animals, but not in the control scars, which were homogeneous in appearance and did not contain any host cardiomyocytes. Transplants of freshly isolated MSCs, cultured MSCs, and 5-azacytidine-treated MSCs all stained positively for cardiac-specific troponin I.

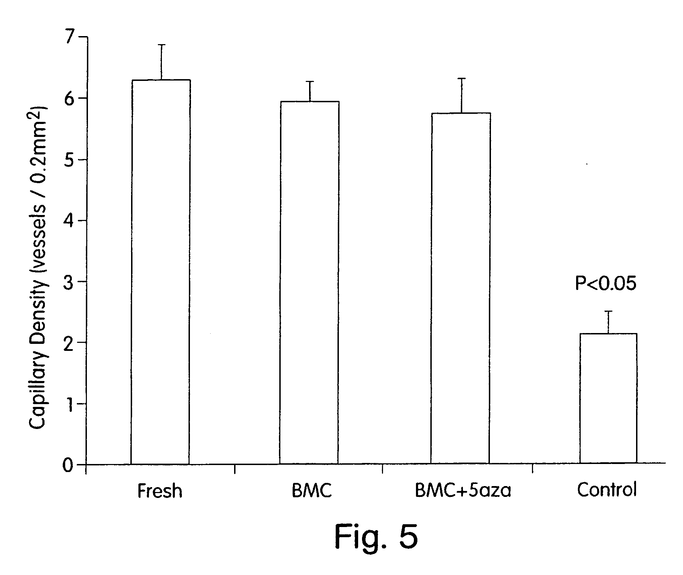

In addition to giving rise to cardiomyocyte-like cells, the transplanted MSCs stimulated angiogenesis. Fig. 5 is a graph showing the capillary densities of transplants of freshly isolated MSCs, cultured MSCs, 5-azacytidine-treated MSCs, and mock transplants (i.e., injection of medium alone). The number of capillaries of the MSC transplanted groups (freshly isolated MSCs: 6.29±0.58; cultured MSCs: 5.93±0.33; MSCs plus 5-aza: 5.74±0.57 vessels/0.2 mm²) was larger (p<0.05) than that of control group (2.12±0.38 vessels/0.2 mm²) (Fig. 5). Some capillary walls were composed of BrdU-positive endothelial cells (Fig. 6). Neither lymphocyte infiltration nor immunorejection was evident. Cartilage, bone and fat did not form in the transplanted area, nor were any tumor-like cells seen.

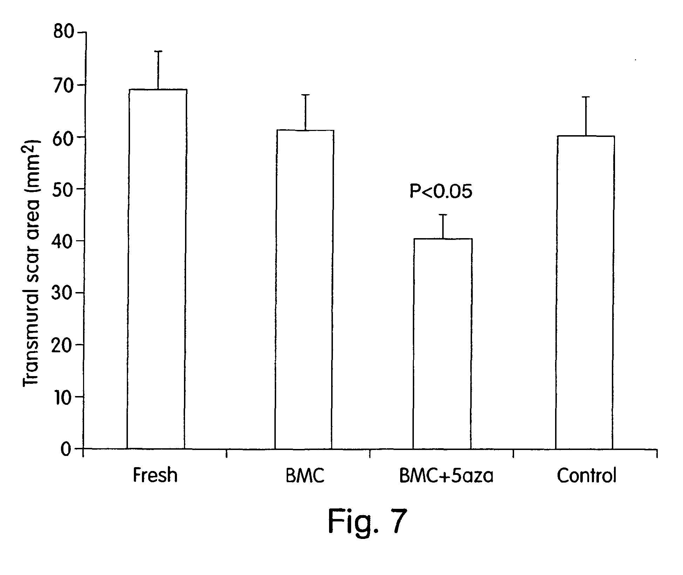

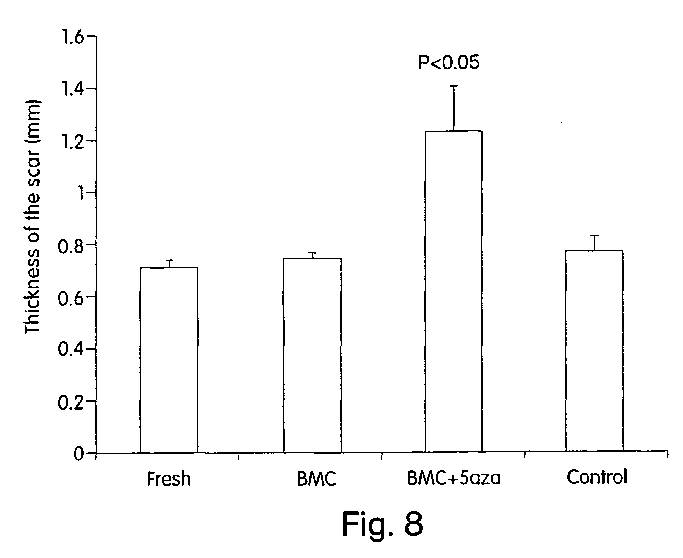

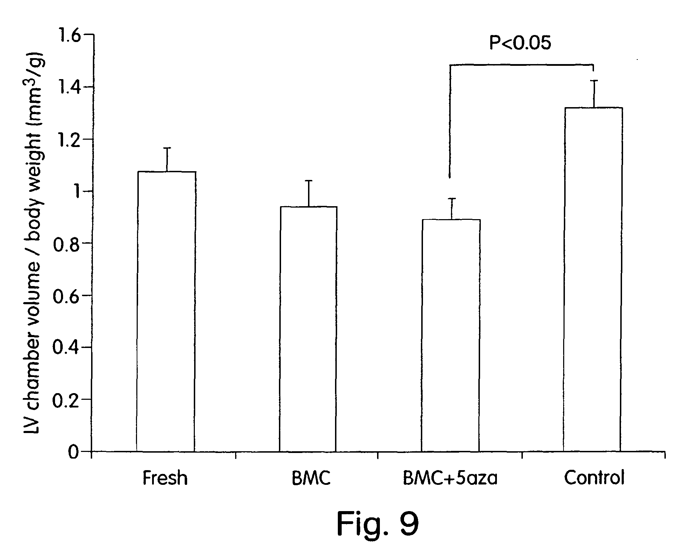

Morphological studies showed that transmural scar area of the 5-aza group was smaller (p<0.05) than the other groups (Fig. 7) and scar thickness of the 5-aza group was larger (p<0.05) than the other groups (Fig. 8). The volume (indicating left ventricular chamber size) of hearts transplanted with 5-azacytidine-treated MSCs was less (p<0.05) than that of the control hearts (Fig. 9). For the above-described morphological studies, the number of rats in each experimental group was N=9 for hearts transplanted with freshly isolated MSCs or cultured MSCs and N=12 for 5-azacytidine-treated and control hearts.

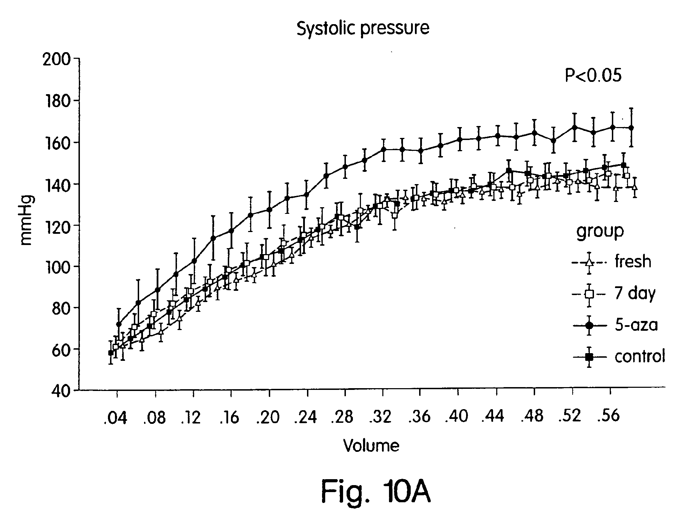

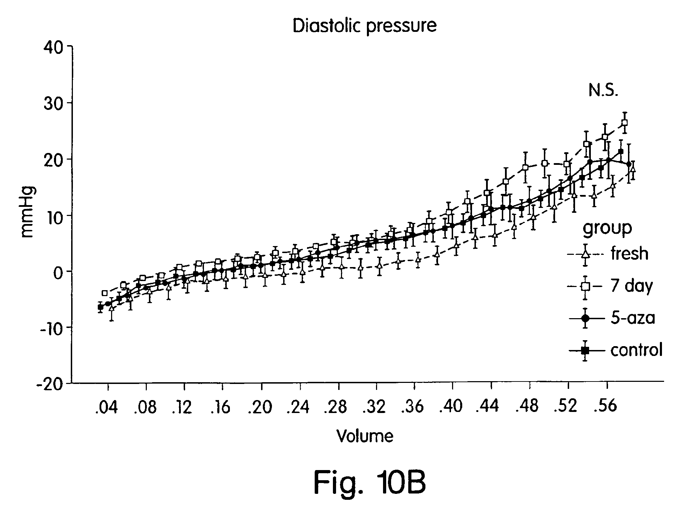

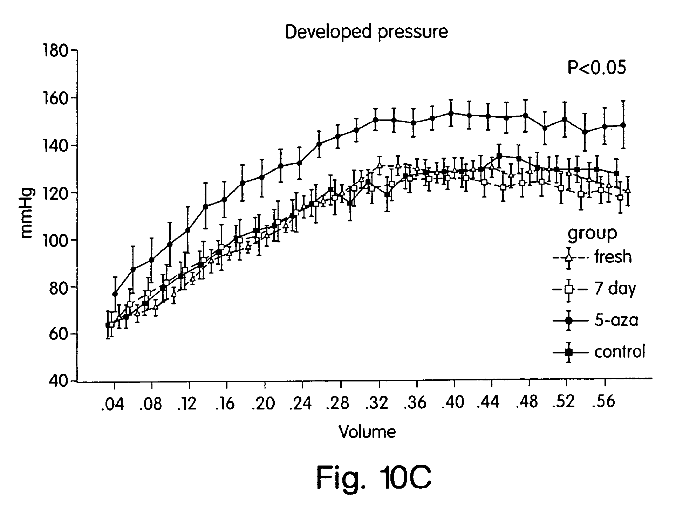

Figs. 10A-10C show, respectively, the systolic, diastolic, and developed pressures of hearts transplanted with freshly isolated MSCs, cultured MSCs, 5-azacytidine-treated MSCs, and mock transplants. Peak systolic and developed pressure of 5-azacytidine-treated MSC transplants were better (p<0.05) than those of the other groups. No difference in function was found among the fresh MSC, cultured MSC, and control (mock) transplants. For the above-described functional studies, the number of rats in each experimental group was N=9 for hearts transplanted with freshly isolated MSCs or cultured MSCs and N=12 for 5-azacytidine-treated and control hearts.

### Conclusions

MSCs prepared from bone marrow cultured with 5-aza can be successfully transplanted into myocardial scar tissue to form cardiac-like tissue. The transplanted cells improved myocardial function compared with the results of the control animals. No immunorejection was observed with the autotransplanted MSCs. Thus, based on the results of the *in vivo* and *in vitro* studies, we conclude that MSCs can be differentiated into cardiomyogenic cells by culturing with 5-aza.

### Example III: Autologous bone marrow cell transplantation into porcine myocardial scar tissue improves myocardial function

Cardiomyogenic differentiation and survival, development of angiogenesis in the transplanted area, and the effect of transplanted cells on infarcted myocardial morphology and function of 5-azacytidine-treated autologous bone marrow cells transplanted into myocardial scar tissue were studied in a swine model of myocardial infarction as described below.

### Methods

### General Surgical Procedures

All surgical procedures performed on the swine were done under general anesthesia. Animals were pre-medicated using ketamine (20-30 mg/kg, intramuscular). Anesthesia was induced using 4% Isoflurane in oxygen (flow rate of 6L/minute). Animals were intubated using a 7.0 mm cuffed ET tube and mechanically ventilated with 100% oxygen to maintain paCO₂ between 35-45 mmHg. Anesthesia was maintained using Isoflurane 1-2.5% in oxygen at a flow rate of 2-3 L/minute and FiO₂ at 1.0.

Electrocardiographic (ECG) electrodes (5 leads) of a bedside monitor were connected to the animal's skin in the standard lead 1 position for the purpose of monitoring the rhythm and rate of the heart during surgery.

### Generation of Myocardial Scars by Coronary Occlusion

A coronary artery occlusion technique was used to infarct the myocardium. The left carotid artery was exposed and a catheter inserted. A coil to cause the coronary artery occlusion was delivered to the left anterior descending coronary artery distal to the first diagonal branch. Systemic blood pressure, heart rate and ECG were monitored for 10 minutes following scar generation. Severe ventricular arrhythmias appearing during this time were treated with intravenous Lidocaine. The left carotid artery was ligated. Subcutaneous tissue and skin was closed with sutures.

Analgesics (3.0 mg Numorphan intramuscularly) and antibiotics were given prior to withdrawal of Isoflurane anesthesia. The animals were recovered from anesthesia in a warm environment and monitored for the first 6 hours postoperatively. Analgesia was given locally every 30 minutes during the first 6 hours and as necessary. An animal technologist monitored the animal's health.

### Isolation of Autologous Bone Marrow Cells and Transplantation into Myocardial Scars

Bone marrow cells were collected by aspiration of the sternum and cultured with 5-aza as described in the previous Examples. The bone marrow aspirate cultured with 5-aza was prepared for transplantation, as described above, immediately prior to transplantation. The cells were suspended in sterile saline at a concentration of 10⁷ cells/ml. Two ml of cell suspension or culture medium were injected into the myocardial scar tissue 4 weeks after coronary artery ligation.

### Analysis of Cardiac Function

To evaluate regional myocardial perfusion and contractile function and to measure global heart function before and after MSC transplantation, 10 mCi of ^{99m}Tc-MIBI (Dupont) was intravenously injected and ^{99m}Tc-Sesamibi Single-Photon Tomography (99m Tc-MIBI SPECT) was done four weeks after coronary artery occlusion (i.e, immediately before transplantation of either 5-aza-treated cultured MSCs or culture medium alone), and again eight weeks after coronary artery occlusion (i.e., four weeks post-transplantation).

Ventricular function of the hearts was measured two days after the second MIBI scan. To measure ventricular function, a small incision was made in the apex of the heart, after which a conductance catheter and a Millar catheter (Model SPC-350) were inserted into the left ventricle. Functional assessment of the left ventricle was performed as described by Li et al. (Ann. Thorac. Surg. 62:654-61, 1996) and Jugdutt and Khan (Circulation 89:2297-2307, 1994).

### Results

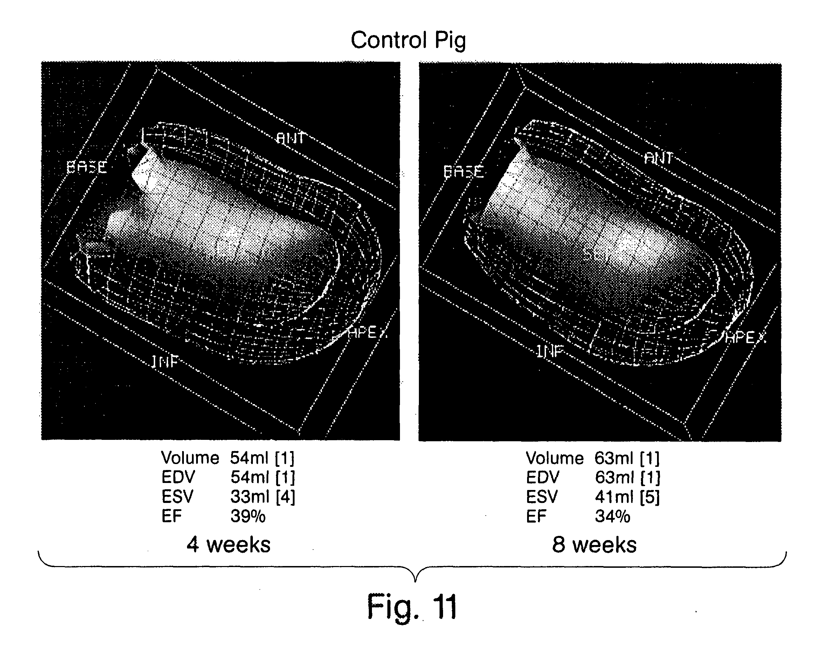

Figs. 11 shows MIBI scans of myocardial function of a control (mock-transplanted) swine heart at 4 weeks (i.e., pre-mock-transplantation) and 8 weeks (i.e., pre-mock-transplantation) after induction of infarction. As illustrated by the MIBI scans, perfusion of the scarred myocardium was decreased and did not improve between 4 and 8 weeks after infarction in the control heart. Myocardial function as measured by ejection fraction decreased in the 4 week period after medium transplantation in the control hearts. The scar expanded during myocardial systole at 4 weeks and at 8 weeks.

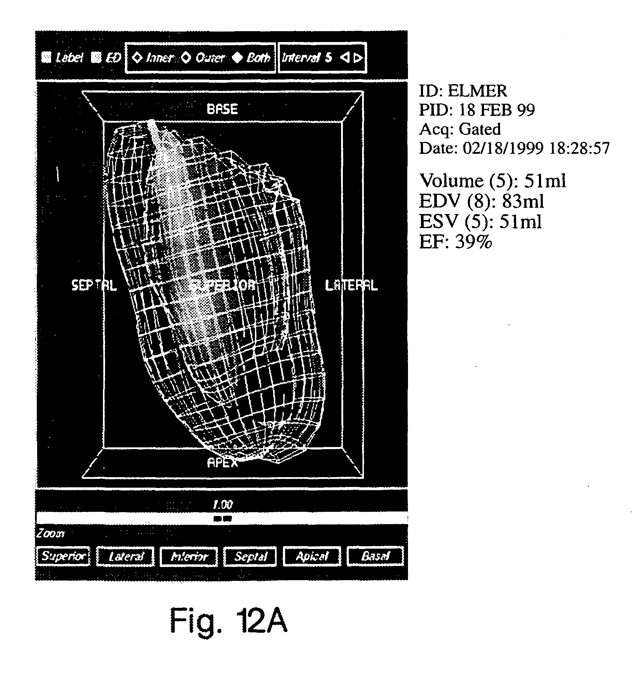

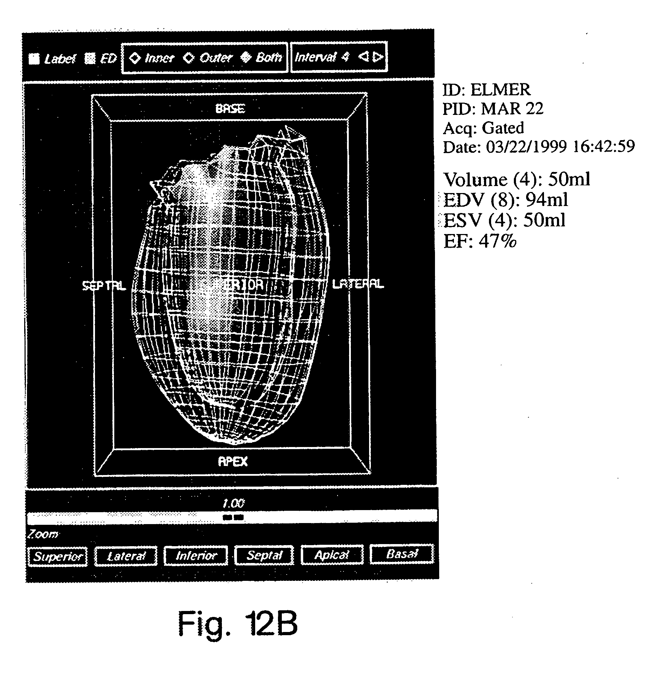

Figs. 12A and 12B shows MIBI scans of myocardial function of an experimental swine heart at 4 weeks (i.e., pre-transplantation; Fig. 12A) and 8 weeks (i.e., post-transplantation; Fig. 12B) after induction of infarction. In contrast to the mock-transplanted control hearts, the animal transplanted with 5-aza-treated cultured bone marrow cells displayed significantly increased angiogenesis in the scar tissue and improved myocardial ejection fraction from 39% at 4 weeks after coronary artery occlusion to 47% at 8 weeks after occlusion. The scar expanded similarly during myocardial systole at 4 weeks in both the transplant and control animals. At 8 weeks there was no expansion during systole of the infarcted region in the bone marrow transplanted heart.

Table 1 shows the cardiac ejection fractions (EF) 4 weeks (pre-transplantation) and 8 weeks (post-transplantation) after coronary artery occlusion in the control and MSC-transplanted pigs described above, plus two additional MSC-transplanted pigs. As expected, all four pigs displayed a below-normal ejection fraction 4 weeks after coronary artery occlusion (ejection fraction for normal hearts is around 50%). However, 4 weeks after transplantation (i.e., 8 weeks after occlusion), the ejection fractions of the three pigs transplanted with 5-aza-treated MSCs were almost normal. In contrast, the ejection fraction of the control, non-transplanted pig had further decreased 8 weeks after occlusion.

**Table 1: Ejection fraction of infarcted hearts before and after transplantation of 5-aza-treated MSCs**

| Material injected | Pre-transptant EF | Post-transplant EF |
|---|---|---|
| 1. culture medium (control) | 39% | 34% |
| 2. 5-aza-MSCs | 39% | 47% |
| 3. 5-aza-MSCs | 33% | 47% |
| 4. 5-aza-MSCs | 45% | 52% |

### Conclusions

Autologous porcine 5-azacytidine-treated bone marrow transplants increased angiogenesis in the coronary artery occlusion porcine model of a myocardial infarction. In contrast, angiogenesis did not occur in the myocardial scars of control hearts. The bone marrow transplant improved myocardial function, as measured by ejection fraction and decreased expansion of the infarcted myocardial wall during systole. No significant improvement in function was found in control hearts. Systolic expansion of the infarcted myocardial wall continued in the control hearts. These results show that implantation of autologous 5-azacytidine-treated bone marrow cells into myocardial scars effectively improves cardiac function.

## Claims

1. Use of a cellular suspension containing mesenchymal stern cells for the preparation of a pharmaceutical composition for treating scarred myocardial tissue, wherein the pharmaceutical composition is to be administered to said scarred myocardial tissue.

2. The use of claim 1, wherein at least one mesenchymal stern cell has been induced to differentiate into a cardiomyogenic cell.

3. The use of claim 1, wherein at least one mesenchymal stern cell is to be integrated into a capillary wall in said scarred myocardial tissue.

4. The use of claim 1, wherein said mesenchymal stern cells have been cultured for at least 7 days.

5. The use of claim 1, wherein said mesenchymal stern cells have been co-cultured with cardiomyocytes.

6. The use of claim 1, wherein said mesenchymal stern cells are autologous.

7. The use of claim 1, wherein said mesenchymal stern cells have been exposed to 5-azacytidine or an analog thereof.

8. The use of claim 7, wherein said 5-azacytidine or said analog thereof is present at a concentration of 1 to 100 *µ*M, preferably 10*µ*m.

9. The use of claim 1, wherein said mesenchymal stern cells are isolated from bone marrow.

10. The use of claim 1, wherein said administering is by injecting.

11. The use of claim 1, wherein said treatment improves cardiac function.

12. The use of claim 1, wherein said mesenchymal stern cells are not passaged.

13. A method of obtaining a population of cells containing cardiomyogenic cells, said method comprising:
a) obtaining mesenchymal stern cells;
b) exposing said mesenchymal stern cells to 5-azacytidine or an analog thereof, wherein said exposing is sufficient to obtain at least one cardiomyogenic cell; and
c) placing said cells from step b) into a medium suitable for injecting the cells into scarred myocardium.

14. The method of claim 13, wherein said mesenchymal stern cells are exposed for at least 7 days.

15. The method of claim 14, wherein the concentration of said 5-azacytidine or said analog thereof is between 1 and 100 µM, preferably 10 µM.

16. The method of claim 13, wherein at least one mesenchymal stern cell differentiates into a cardiomyogenic cell.

17. The method of claim 13, wherein at least one mesenchymal stern cell differentiates into an endothelial cell.

18. The method of claim 13, wherein said mesenchymal stern cells are isolated from bone marrow.

19. The method of claim 13, wherein said mesenchymal stern cells are not passaged.

## Patentansprüche

1. Verwendung einer Zellsuspension, die mesenchymale Stammzellen enthält, zur Herstellung einer pharmazeutischen Zusammensetzung, zur Behandlung vernarbten myokardinalen Gewebes, wobei die pharmazeutische Zusammensetzung dem vernarbten myokardinalen Gewebe verabreicht werden muss.

2. Verwendung nach Anspruch 1, wobei mindestens eine mesenchymale Stammzelle induziert wurde, um zu einer kardiomyogenen Zelle zu differenzieren.

3. Verwendung nach Anspruch 1, wobei mindestens eine mesenchymale Stammzelle in eine Kapillarwand in dem vernarbten myokardinalen Gewebe integriert werden muss.

4. Verwendung nach Anspruch 1, wobei die mesenchymalen Stammzellen mindestens 7 Tage lang kultiviert wurden.

5. Verwendung nach Anspruch 1, wobei die mesenchymalen Stammzellen mit Kardiomyocyten cokultiviert wurden.

6. Verwendung nach Anspruch 1, wobei die mesenchymalen Stammzellen autolog sind.

7. Verwendung nach Anspruch 1, wobei die mesenchymalen Stammzellen 5-Azacytidin oder einem Analogon davon ausgesetzt wurden.

8. Verwendung nach Anspruch 7, wobei das 5-Azacytidin oder das Analogon davon in einer Konzentration von 1 bis 100 µM, bevorzugt von 10 µM, vorliegt.

9. Verwendung nach Anspruch 1, wobei die mesenchymalen Stammzellen aus Knochenmark isoliert sind.

10. Verwendung nach Anspruch 1, wobei das Verabreichen durch Injizieren erfolgt.

11. Verwendung nach Anspruch 1, wobei die Behandlung die Herzfunktion verbessert.

12. Verwendung nach Anspruch 1, wobei keine Passage der mesenchymalen Stammzellen erfolgt.

13. Verfahren zum Erhalten einer Population von Zellen, die kardiomyogene Zellen enthält, wobei das Verfahren umfasst:
a) Erhalten mesenchymaler Stammzellen;
b) Aussetzen der mesenchymalen Stammzellen gegenüber 5-Azocytidin oder einem Analogon davon, wobei das Aussetzen hinreichend ist, um mindestens eine kardiomygene Zelle zu erhalten; und
c) platzieren der Zellen aus Schritt b) in ein Medium, das geeignet ist, um die Zellen in vernarbtes Myokardium zu injizieren.

14. Verfahren nach Anspruch 13, wobei die mesenchymalen Stammzellen mindestens 7 Tage ausgesetzt werden.

15. Verfahren nach Anspruch 14, wobei die Konzentration des 5-Azacytidins oder des Analogons davon zwischen 1 und 100 µM, bevorzugt 10 µM, beträgt.

16. Verfahren nach Anspruch 13, wobei mindestens eine mesenchymale Stammzelle zu einer kardiomyogenen Zelle differenziert.

17. Verfahren nach Anspruch 13, wobei mindestens eine mesenchymale Stammzelle zu einer endothelialen Zelle differenziert.

18. Verfahren nach Anspruch 13, wobei die mesenchymalen Stammzellen aus Knochenmark isoliert werden.

19. Verwendung nach Anspruch 13, wobei keine Passage der mesenchymalen Stammzellen erfolgt.

## Revendications

1. Utilisation d'une suspension cellulaire contenant des cellules souches mésenchymateuses pour la préparation d'une composition pharmaceutique pour traiter un tissu myocardique portant des cicatrices, où la composition pharmaceutique est destinée à être administrée audit tissu myocardique portant des cicatrices.

2. Utilisation selon la revendication 1 où au moins une cellule souche mésenchymateuse a été amenée à se différencier en une cellule cardiomyogène.

3. Utilisation selon la revendication 1 où au moins une cellule souche mésenchymateuse est destinée à être intégrée dans une paroi capillaire dans ledit tissu myocardique portant des cicatrices.

4. Utilisation selon la revendication 1 où lesdites cellules souches mésenchymateuses ont été cultivées pendant au moins 7 jours.

5. Utilisation selon la revendication 1 où lesdites cellules souches mésenchymateuses ont été co-cultivées avec des cardiomyocytes.

6. Utilisation selon la revendication 1 où lesdites cellules souches mésenchymateuses sont autologues.

7. Utilisation selon la revendication 1 où lesdites cellules souches mésenchymateuses ont été exposées à la 5-azacytidine ou un analogue de celle-ci.

8. Utilisation selon la revendication 7 où ladite 5-azacytidine ou ledit analogue de celle-ci est présente à une concentration de 1 à 100 µM, de préférence de 10 µM.

9. Utilisation selon la revendication 1 où lesdites cellules souches mésenchymateuses sont isolées à partir de moelle osseuse.

10. Utilisation selon la revendication 1 où ladite administration est par injection.

11. Utilisation selon la revendication 1 où ledit traitement améliore la fonction cardiaque.

12. Utilisation selon la revendication 1 où lesdites cellules souches mésenchymateuses ne sont pas soumises à des passages.

13. Procédé d'obtention d'une population de cellules contenant des cellules cardiomyogènes, ledit procédé comprenant :
a) l'obtention de cellules souches mésenchymateuses ;
b) l'exposition desdites cellules souches mésenchymateuses à la 5-azacytidine ou un analogue de celle-ci, où ladite exposition est suffisante pour obtenir au moins une cellule cardiomyogène ; et
c) la mise en place desdites cellules provenant de l'étape b) dans un milieu approprié pour injecter les cellules dans un myocarde portant des cicatrices.

14. Procédé selon la revendication 13 où lesdites cellules souches mésenchymateuses sont exposées pendant au moins 7 jours.

15. Procédé selon la revendication 14 où la concentration de ladite 5-azacytidine ou dudit analogue de celle-ci est entre 1 et 100 µM, de préférence 10 µM.

16. Procédé selon la revendication 13 où au moins une cellule souche mésenchymateuse se différencie en une cellule cardiomyogène.

17. Procédé selon la revendication 13 où au moins une cellule souche mésenchymateuse se différencie en une cellule endothéliale.

18. Procédé selon la revendication 13 où lesdites cellules souches mésenchymateuses sont isolées à partir de moelle osseuse.

19. Procédé selon la revendication 13 où lesdites cellules souches mésenchymateuses ne sont pas soumises à des passages.
